# EUROPEAN PATENT APPLICATION

(11) **EP 4 000 623 A1**
(43) Date of publication of application: **25.05.2022**
(21) Application number: 20382973.4
(22) Date of filing: 11.11.2020
(51) Int. Cl.: A61K 31/496, A61P 31/14

(54) **5-(2-(1H-INDOL-3-YL))-ETHYL)- PIPERAZIN-2-ONE DERIVATIVES FOR USE IN THE TREATMENT OF VIRAL INFECTIONS BY VIRUSES OF THE FAMILY CORONAVIRIDAE**

(71) Applicant: Consejo Superior De Investigaciones Científicas, 28006 Madrid (ES)
(72) Inventor: GUTIÉRREZ RODRÍGUEZ, Marta, E-28006 Madrid (ES); HERRANZ HERRANZ, Rosario, E-28006 Madrid (ES); GASTAMINZA LANDART, Pablo, E-28006 Madrid (ES); GARAIGORTA DE DIOS, Urtzi, E-28006 Madrid (ES)
(74) Representative: ABG Intellectual Property Law, S.L.

(57) **Abstract**

The present invention relates to new derivatives of 5-(2-(1*H*-indol-3-yl)ethyl-2-piperazin-2-one or pharmaceutically acceptable salts thereof and to combinations of said compounds with other active ingredients, for use in the treatment and/or prevention of viral infections by viruses from the family *Coronaviridae,* to the use of said compound or its combinations in the manufacture of a medicament for the treatment or prevention of said diseases and to a method of treating and/or preventing said diseases by administration of said compound or its combinations.

## Description

### FIELD OF THE INVENTION

The present invention relates to compounds for use in the treatment and/or prevention of viral infections by viruses of the family *Coronaviridae,* especially by respiratory syndrome-related coronaviruses selected from the species *"Middle East respiratory syndrome-related coronavirus"* (such as MERS-CoV) and *"Severe respiratory syndrome-related coronavirus"* (such as SARS-CoV and SARS-CoV-2).

### BAKGROUND OF THE INVENTION

The viruses of the family *Coronaviridae* are enveloped viruses with a positive-sense single-stranded RNA genome and a nucleocapsid of helical symmetry. The name of the family is derived from the Latin corona, meaning "crown" or "halo", which refers to the characteristic appearance reminiscent of a solar corona around the virions (virus particles) when viewed under two-dimensional transmission electron microscopy, due to the surface being covered in club-shaped protein spikes.

In December 2019 a new infectious disease caused by severe acute respiratory syndrome coronavirus 2 (SARS-CoV-2) was first identified in Wuhan, the capital of China's Hubei province, and has since then spread globally, resulting in the ongoing 2019-20 coronavirus pandemic. The disease has been given the name Coronavirus disease 2019 or COVID-19. This disease has evolved into an appalling global pandemic worldwide.

The etiological agent of this severe respiratory syndrome is caused by a coronavirus, named SARS-CoV-2 due to its genetic similarity to the Severe Acute Respiratory Syndrome coronavirus (SARS-CoV) that emerged in 2003 as a limited epidemic in different areas of southeast Asia.

SARS-CoV-2 replicates efficiently in asymptomatic patients, which are largely responsible for the spread of the virus worldwide, given that high replication levels are not necessarily associated with clinical manifestations of the disease that may restrict infected patient mobility. This characteristic and the prolonged periods of time of productive infection have definitely contributed to the establishment of the current pandemic. While silent in a majority of cases, SARS-CoV-2 may lead to COVID-19, which is primarily but not exclusively characterized by severe pneumonia, that may cause death in fragile segments of the population, notably, but not exclusively in the elderly.

Common symptoms include fever, cough, and shortness of breath. Other symptoms may include muscle pain, sputum production, diarrhea, sore throat, loss of smell, and abdominal pain. While the majority of cases result in mild symptoms, some progress to bilateral pneumonia and multi-organ failure.

Early immunological studies as well as prior experience with SARS-CoV suggest that a prophylactic vaccine may be developed against SARS-CoV-2. Given the severity and wide impact of the disease, numerous efforts are being put into the development of such vaccine. While this is certainly the best approach to contain future outbreaks, it is clear that additional strategies need to be in place for patients that already acquired the infection. Antiviral treatment may contribute to infection resolution and better prognosis of hospitalized patients. Eventually, antivirals may also be considered under certain circumstances as prophylactic treatment option for the containment of specific outbreaks.

Considering that the only approved antiviral drug against SARS-CoV-2, remdesivir, is non-specific, injectable and has limited efficacy at improving COVID-19 clinical outcome, there is an urgent need for the identification of novel antivirals against SARS-CoV-2.

Given the urgency of the matter, a large effort has been done in drug repurposing, with a long list of repurposing candidates currently under clinical evaluation.

In addition to this urgent effort, it is of capital importance the discovery of new antivirals, which could allow the identification of clinical candidates. This should be the way to arrive in medium/long term to efficient drugs for COVID-19 treatment, and potentially also for other coronavirus infections that could emerge. With this aim, the inventors have selected and screened various small libraries of compounds from in-house collections as potential agents for the treatment and/or prevention of viral infections by viruses from the family Coronaviridae.

### SUMMARY OF THE INVENTION

The inventors have surprisingly found that certain derivatives of 5-(2-(1*H*-indol-3-yl)ethyl-2-piperazin-2-one are useful in the treatment and/or prevention of viral infections by virus from the family *Coronaviridae.*
Thus, in a first aspect, the present invention relates to compounds of formula (I) 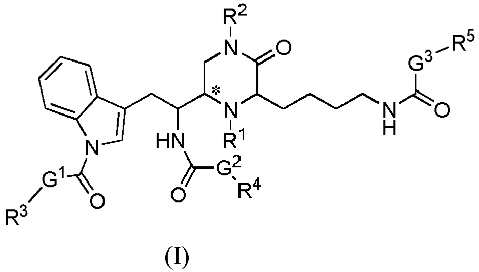 its stereoisomers and pharmaceutically acceptable salts thereof for use in the treatment and/or prevention of an infection by viruses of the family *Coronaviridae,* wherein
- R¹ is a group selected from hydrogen, benzyl and -CH₂C(O)O-R⁶,
- R² is a group selected from hydrogen and benzyl,
- R³, R⁴ and R⁵ are independently selected from the group consisting of linear or branched C₁₋₆-alkyl, 6-14 membered aryl, 6-14 membered heteroaryl comprising 1, 2 or 3 heteroatoms selected from the group consisting of N, O and S, wherein the aryl and heteroaryl groups may be substituted by 1 to 3 substituents selected from the group consisting of halogen atom, linear or branched C₁₋₃ haloalkyl, linear or branched C₁₋₃ alkyl, linear or branched C₁₋₃ alkoxy, cyano group and hydroxy group,
- G¹, G² and G³ are either absent or are independently selected from the group consisting of -O-, -O-CH₂-, wherein the oxygen atom is bound to the corresponding carbonyl group, -CH₂-, and -NH-; and.
- R⁶ is selected from the group consisting of linear or branched C₁₋₆-alkyl and benzyl.

In a second aspect, the invention relates to the use of a compound as defined in the first aspect in the manufacture of a medicament for the treatment and/or prevention of viral infections by viruses of the family *Coronaviridae,* especially by the respiratory syndrome-related coronaviruses selected from the species *"Middle East respiratory syndrome-related coronavirus"* (such as MERS-CoV) and *"Severe respiratory syndrome-related coronavirus"* (such as SARS-CoV and SARS-CoV-2).

In a third aspect, the invention also relates to a method of treating and/or preventing viral infections by viruses of the family *Coronaviridae,* especially by respiratory syndrome-related coronaviruses selected from the species *"Middle East respiratory syndrome-related coronavirus"* (such MERS-CoV) and *"Severe respiratory syndrome-related coronavirus"* (such as SARS-CoV and SARS-CoV-2), in a subject, comprising administering to said subject a therapeutically effective amount of a compound as defined in the first aspect of the invention.

In a fourth aspect, the present invention relates to a combination comprising one or more compounds as defined in the first aspect of the invention and or more additional compounds useful in the treatment of viral infections by viruses of the family *Coronaviridae.* Some compounds that have been disclosed as potentially useful are remdesivir, ribavirin, favipiravir, chloroquine, hydroxychloroquine, lopinavir, ritonavir, umifenovir and recombinant type I interferon.

In a fifth aspect, the present invention relates to a pharmaceutical composition comprising a compound of formula (I) and at least one pharmaceutically acceptable excipient for use in the treatment and/or prevention of viral infections by viruses of the family *Coronaviridae.*

### DESCRIPTION OF THE INVENTION

In the first aspect, the present invention relates to a compound of formula (I) its stereoisomers and pharmaceutically acceptable salts thereof for use in the treatment and/or prevention of an infection by viruses of the family *Coronaviridae,* 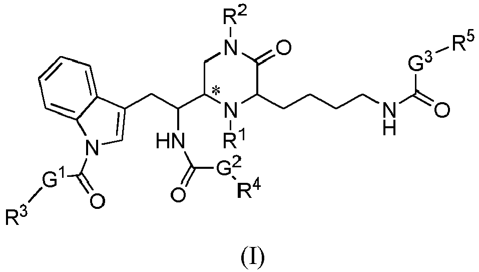 wherein
- R¹ is a group selected from hydrogen, benzyl and -CH₂C(O)O- R⁶,
- R² is a group selected from hydrogen and benzyl,
- R³, R⁴ and R⁵ are independently selected from the group consisting of linear or branched C₁₋₆-alkyl, 6-14 membered aryl, 6-14 membered heteroaryl comprising 1, 2 or 3 heteroatoms selected from the group consisting of N, O and S, wherein the aryl and heteroaryl groups may be substituted by 1 to 3 substituents selected from the group consisting of halogen atom, linear or branched C₁₋₃ haloalkyl, linear or branched C₁₋₃ alkyl, linear or branched C₁₋₃ alkoxy, cyano group and hydroxy group,
- G¹, G² and G³ are either absent or are independently selected from the group consisting of -O-, -O-CH₂-, wherein the oxygen atom is bound to the corresponding carbonyl group, -CH₂-, and -NH-; and.
- R⁶ is selected from the group consisting of linear or branched C₁₋₆-alkyl and benzyl.

The invention also relates to the use of a compound of formula (I) in the manufacture of a medicament for the treatment and/or prevention of viral infections by viruses of the family *Coronaviridae,* especially by respiratory syndrome-related coronaviruses selected from the species *"Middle East respiratory syndrome-related coronavirus"* (such as MERS-CoV) and *"Severe respiratory syndrome-related coronavirus"* (such SARS-CoV and SARS-CoV-2).

The invention also relates to a method of treating and/or preventing viral infections by viruses of the family *Coronaviridae,* especially by respiratory syndrome-related coronaviruses selected from the species *"Middle East respiratory syndrome-related coronavirus"* (such as MERS-CoV) and *"Severe respiratory syndrome-related coronavirus"* (such as SARS-CoV and SARS-CoV-2) in a subject, comprising administering to said subject a therapeutically effective amount of a compound of formula (I).

The term "viruses of the family *Coronaviridae",* as used herein, is used to designate any viral species of the taxonomic family *"Coronaviridae"* including those of the genera *"Alphaletovirus", "Alphacoronavirus", "Betacoronavirus", "Gammacoronavirus"* and *"Deltacoronavirus".*

In a preferred embodiment of the present invention, the viruses are selected from betacoronaviruses, in particular from the lineage *"Sarbecovirus"* and still more preferably from the species *"Middle East respiratory syndrome-related coronavirus"* (such as MERS-CoV) and *"Severe respiratory syndrome-related coronavirus"* (such as SARS-CoV and SARS-CoV-2).

The terms "treating" and "treatment", as used herein, means reversing, alleviating, inhibiting the progress of, the disease or condition to which such term applies, or one or more symptoms of such disease or condition, such as lowering the viral load in a patient with respect to pretreatment levels.

The terms "preventing" and "prevention", as used herein, means avoiding or inhibiting the onset of one or more symptoms of coronavirus infections such as fever, cough, shortness of breath, muscle pain, sputum production, diarrhea, sore throat, loss of smell, pneumonia and abdominal pain.

Preferably, the compounds disclosed herein, are used for the treatment and/or prevention of viral infections by *"Severe respiratory syndrome-related coronavirus"* and most preferably for the treatment and/or prevention of viral infections by SARS-CoV-2.

In a particular embodiment of the first, second and third aspects of the invention in the compound for use of formula (I) two of the groups R³, R⁴ and R⁵ are identical.

In a particular embodiment of the first, second and third aspects of the invention in the compound for use of formula (I) two of the groups G¹, G² and G³ are identical.

In a particular embodiment of the first, second and third aspects of the invention in the compound for use of formula (I) R³-G¹-, R⁴-G²- and R⁵-G³- are independently selected from the group consisting of C₁₋₆-alkoxy and C₆₋₁₄-aryl-CH₂-O- and C₆₋₁₄-aryl-CH₂-.

In a particular embodiment of the first, second and third aspects of the invention in the compound for use of formula (I) R³-G¹-, R⁴-G²- and R⁵-G³- are independently selected from the group consisting *tert*-butoxy and fluorenylmethyloxy.

In a particular embodiment of the first, second and third aspects of the invention in the compound for use of formula (I) R² is hydrogen.

In a particular embodiment of the first, second and third aspects of the invention the compound for use of formula (I) is selected from the group consisting of:
- (5*R*,3*S*)-5-((*S*)-1-(*tert*-butoxycarbonyl)amino-2-(1-(*tert*-butoxycarbonyl)(indol-3-yl))-ethyl)-3-(4-(*tert*-butoxycarbonyl)amino-butyl)-2-oxopiperazine
- (5*S*,3*S*)-5-((*S*)-1-(*tert*-butoxycarbonyl)amino-2-(1-(*tert*-butoxycarbonyl)(indol-3-yl))-ethyl)-3-(4-(*tert*-butoxycarbonyl)amino-butyl)-2-oxopiperazine
- (5*R*,3*S*)-4-benzyl-5-((*S*)-1-(*tert*-butoxycarbonyl)amino-2-(1-(*tert-*butoxycarbonyl)(indol-3-yl))-ethyl)-3-(4-(*tert*-butoxycarbonyl)amino-butyl)-2-oxopiperazine
- (5*S*,3*S*)-4-benzyl-5-((*S*)-1-(*tert*-butoxycarbonyl)amino-2-(1-(*tert-*butoxycarbonyl)(indol-3-yl))-ethyl)-3-(4-(*tert*-butoxycarbonyl)amino-butyl)-2-oxopiperazine
- (5*R*,3*S*)-5-((*S*)-1-(*tert*-butoxycarbonyl)amino-2-(1-(*tert*-butoxycarbonyl)(indol-3-yl))-ethyl)-4-(*tert*-butoxycarbonyl)methyl-3-(4-(*tert*-butoxycarbonyl)aminobutyl)-2-oxopiperazine
- (5*S*,3*S*)-5-((*S*)-1-(*tert*-butoxycarbonyl)amino-2-(1-(*tert*-butoxycarbonyl)(indol-3-yl))-ethyl)-4(*tert*-butoxycarbonyl)methyl-3-(4-(*tert*-butoxycarbonyl)aminobutyl)-2-oxopiperazine
- (5*R*,3*S*)-1,4-dibenzyl-5-((*S*)-1-(*tert-*butoxycarbonyl)amino-2-(1-(*tert-*butoxycarbonyl)(indol-3-yl))-ethyl)-3-(4-(*tert-*butoxycarbonyl)amino-butyl)-2-oxopiperazine
- (5*S*,3*S*)-1,4-dibenzyl-5-((*S*)-1-(*tert*-butoxycarbonyl)amino-2-(1-(*tert-*butoxycarbonyl)(indol-3-yl))-ethyl)-3-(4-(*tert*-butoxycarbonyl)amino-butyl)-2-oxopiperazine
- (5*R*,3*S*)-1-benzyl-5-((*S*)-1-(*tert*-butoxycarbonyl)amino-2-(1-(*tert-*butoxycarbonyl)(indol-3-yl))-ethyl)-4-(*tert*-butoxycarbonyl)methyl-3-(4-(*tert-*butoxycarbonyl)amino-butyl)-2-oxopiperazine
- (5*S*,3*S*)-1-benzyl-5-((*S*)-1-(*tert*-butoxycarbonyl)amino-2-(1-(*tert-*butoxycarbonyl)(indol-3-yl))-ethyl)-4-(*tert*-butoxycarbonyl)methyl-3-(4-(*tert-*butoxycarbonyl)amino-butyl)-2-oxopiperazine
and pharmaceutically acceptable salts thereof.

As used herein the term alkyl is used to designate linear or branched hydrocarbon radicals (CₙH₂ₙ₊₁). Examples include methyl, ethyl, *n*-propyl, *i*-propyl, *n*-butyl, *sec-*butyl, *tert*-butyl, *n*-pentyl, 1-methyl-butyl, 2-methyl-butyl, isopentyl, 1-ethylpropyl, 1,1-dimethylpropyl, 1,2-dimethylpropyl, *n*-hexyl, 1-ethylbutyl, 2-ethylbutyl, 1,1-dimethylbutyl, 1,2-dimethylbutyl, 1,3-dimethylbutyl, 2,2-dimethylbutyl, 2,3-dimethylbutyl, 2-methylpentyl and 3-methylpentyl. The term Cₙ₋ₘ alkyl is therefore to be understood as designating an alkyl comprising n to m carbon atoms

As used herein the term Cₙ₋ₘ alkoxy is used to designate radicals which contain a linear or branched Cₙ₋ₘ alkyl group linked to an oxygen atom. Preferred alkoxy radicals include methoxy, ethoxy, *n*-propoxy, *i*-propoxy, *n*-butoxy, *sec-*butoxy and *tert*-butoxy.

As used herein, the term n-m membered aryl is used to designate unsaturated aromatic carbocyclic ring systems having n to m members in the ring system. Said radicals may optionally be substituted by 1 or 2 groups selected from halogen atom, linear or branched C₁₋₃ haloalkyl, linear or branched C₁₋₃ alkyl, linear or branched C₁₋₃ alkoxy, cyano group and hydroxy group. When an aryl radical carries 2 or more substituents, the substituents may be the same or different. Preferred aryl groups are phenyl and fluorenyl.

As used herein, the term n-m membered heteroaryl is used to designate unsaturated aromatic ring systems having n to m members in the ring system selected from the group consisting of C, N, O and S wherein at least one of the members is one of N, O, and S. Said radicals may optionally be substituted by 1 or 2 groups selected from halogen atom, linear or branched C₁₋₃ haloalkyl, linear or branched C₁₋₃ alkyl, linear or branched C₁₋₃ alkoxy, cyano group and hydroxy group. When a heteroaryl radical carries 2 or more substituents, the substituents may be the same or different.

As used herein, the term "benzyl" refers to the group phenyl-CH₂-.

As used herein, the term "*tert-*butoxycarbonyl" refers to the group (CH₃)₃C-O-C(=O)-.

The term "pharmaceutically acceptable" refers to molecular entities and compositions that are physiologically tolerable and do not typically produce an allergic or similar untoward reaction, such as gastric upset, dizziness and the like, when administered to a human. Preferably, as used herein, the term "pharmaceutically acceptable" means approved by a regulatory agency of the Federal or a state government or listed in the U.S. or European Pharmacopeiae or other generally recognized pharmacopeia for use in animals, and more particularly in humans.

Further, the term "pharmaceutically acceptable salt" refers to any salt, which, upon administration to the recipient is capable of providing (directly or indirectly) a compound as described herein. For instance, a pharmaceutically acceptable salt of compounds provided herein may be acid addition salts, base addition salts or metallic salts, and they can be synthesized from the parent compound, which contains a basic or acidic moiety by conventional chemical methods. Generally, such salts are, for example, prepared by reacting the free acid or base forms of these compounds with a stoichiometric amount of the appropriate base or acid in water or in an organic solvent or in a mixture of the two. Generally, non-aqueous media like ether, ethyl acetate, ethanol, isopropanol or acetonitrile are preferred. Examples of the acid addition salts include mineral acid addition salts such as, for example, hydrochloride, hydrobromide, hydroiodide, sulphate, nitrate, phosphate, and organic acid addition salts such as, for example, acetate, maleate, fumarate, citrate, oxalate, succinate, tartrate, malate, mandelate, methanesulphonate and p-toluenesulphonate. Examples of the alkali addition salts include inorganic salts such as, for example, ammonium, and organic alkali salts such as, for example, ethylenediamine, ethanolamine, *N,N*-dialkylenethanolamine, triethanolamine, glucamine and basic amino acid salts. Examples of the metallic salts include, for example, sodium, potassium, calcium, magnesium, aluminium and lithium salts.

In another particular embodiment, the viral infection is an infection by *"Severe respiratory syndrome-related coronavirus"* and most preferably by SARS-CoV-2.

The compounds for use according to the invention may be administered by any appropriate route (via), such as, oral (e.g., oral, sublingual, etc.), parenteral (e.g., subcutaneous, intramuscular, intravenous, etc.), vaginal, rectal, nasal, topical, ophtalmic, inhaled, intranasal, intratracheal, pulmonary, etc., preferably oral, inhaled or parenteral, more preferably intravenous.

The compounds for use according to the invention may, in particular, be administered by inhalation. In this case, they are advantageously formulated as dry powder compositions and may, for example, be presented in capsules and cartridges of for example gelatine or blisters of for example laminated aluminium foil, for use in an inhaler or insufflator. Formulations generally contain a powder mix for inhalation of the compound of the invention and a suitable powder base (carrier substance) such as lactose or starch. Use of lactose is preferred.

In particular, the compounds for use according to the invention are administered as a pharmaceutical composition, which comprises the corresponding (active) compound and one or more pharmaceutically acceptable excipients.

The term "pharmaceutically acceptable excipient" refers to a vehicle, diluent, or adjuvant that is administered with the active ingredient. Such pharmaceutical excipients can be sterile liquids, such as water and oils, including those of petroleum, animal, vegetable, or synthetic origin, such as peanut oil, soybean oil, mineral oil, sesame oil, and similars. Water or saline aqueous solutions and aqueous dextrose and glycerol solutions, particularly for injectable solutions, are preferably used as vehicles. Suitable pharmaceutical vehicles are known by the skilled person.

The pharmaceutically acceptable excipient necessary to manufacture the desired pharmaceutical composition of the invention will depend, among other factors, on the elected administration route. Said pharmaceutical compositions may be manufactured according to conventional methods known by the skilled person in the art.

The compounds for use according to the invention may be administered in a "therapeutically effective amount", i.e. a nontoxic but sufficient amount of the corresponding compound to provide the desired effect. The amount that is "effective" will vary from subject to subject, depending on the age and general condition of the individual, the particular compound administered, and the like. Thus, it is not always possible to specify an exact "therapeutically effective amount". However, an appropriate amount in any individual case may be determined by one of ordinary skill in the art using routine experimentation.

The compounds for use according to the invention will typically be administered once or more times a day, for example 1, 2, 3 or 4 times daily, with typical total daily doses depending on the particular compound and severity of the disease, and may be easily determined by the skilled practitioner.

By way of example, typical total daily doses the compound of the invention are in the range of from 0.1 to 2000 mg/day, preferably from 1 to 600 mg/ day, even more preferably from 1 to 100 mg/day.

The pharmaceutical compositions may be prepared using standard methods such as those described or referred to in the Spanish, European and US Pharmacopoeias and similar reference texts.

The term "subject" refers to a mammal, e.g., a human.

The compounds for use according to the invention may be administered as the sole active ingredient or in combination with other active ingredients. In a particular embodiment, the compounds are used as the sole active ingredient. In another particular embodiment, the compounds are used in combination with other active ingredients.

In another aspect, the present invention relates to a combination comprising one or more compounds selected useful in the treatment of viral infections by virus of the family *Coronaviridae.* Some compounds that have been disclosed as potentially useful are selected from the group consisting of remdesivir, ribavirin, favipiravir, chloroquine, hydroxychloroquine, lopinavir, ritonavir, umifenovir and, recombinant type I interferon,

The invention also relates to the use of a combination comprising one or more compounds useful in the treatment of viral infections by virus of the family *Coronaviridae.* Some compounds that have been disclosed as potentially useful are selected from the group consisting of remdesivir, ribavirin, favipiravir, chloroquine, hydroxychloroquine, lopinavir, ritonavir, umifenovir and, recombinant type I interferon.

The invention also relates a method of treating and/or preventing viral infections by coronavirus, especially by respiratory syndrome-related coronavirus selected from the species *"Middle East respiratory syndrome-related coronavirus"* (such as MERS-CoV) and *"Severe respiratory syndrome-related coronavirus"* (such as SARS-CoV and SARS-CoV-2) in a subject, comprising administering to said subject a therapeutically effective amount of a combination comprising one or more compounds selected from the group consisting of remdesivir, ribavirin, favipiravir, chloroquine, hydroxychloroquine, lopinavir, ritonavir, umifenovir and, recombinant type I interferon.

The term "combination" refers to a product comprising one or more of the defined compounds, either in a single composition or in several compositions (or units), in which case the corresponding compounds are distributed among the several compositions. Preferably, the combination refers to several compositions, in particular comprising one composition (or unit) per compound (compound as defined above) of the combination. The expression "one or more" when characterizing the combination refers to at least one, preferably 1, 2, 3, 4, or 5 compounds, more preferably, 1, 2 or 3 compounds, even more preferably 1 or 2 compounds.

When the combination is in the form of a single composition, the compounds present in the combination are always administered simultaneously.

When the combination is in the form of several compositions (or units), each of them having at least one of the compounds of the combination, the compositions or (units) may be administered simultaneously, sequentially or separately.

Simultaneous administration means that the compounds or compositions (or units) are administered at the same time.

Sequential administration means that the compounds or compositions (or units) are administered at different time points in a chronologically staggered manner.

Separate administration means that the compounds or compositions (or units) are administered at different time points independently of each other.

In particular, the combinations for use according to the invention are administered as pharmaceutical compositions, which comprise the corresponding (active) compounds and a pharmaceutically acceptable excipient, as previously defined.

The combinations for use according to the invention will typically be administered once or more times a day, for example 1, 2, 3 or 4 times daily, with typical total daily doses depending on the particular compound and severity of the disease, and may be easily determined by the skilled practitioner.

In another particular embodiment, the viral infection is an infection by SARS-CoV-2 virus.

The following examples represent specific embodiments of the present invention. They do not intend to limit in any way the scope of the invention defined in the present description.

### Synthesis of the compounds.

The compounds of general formula (Ia) of the present invention wherein both R¹ and R² are hydrogen atoms and R³-G¹-CO-, R⁴-G²-CO- and R⁵-G³-CO- are either *tert-*butoxycarbonyl or fluorenylmethyloxycarbonyl groups (designated as R-CO- in the following schemes) can be synthesized as described in Valdivielso, Á. M. et al. Eur. J. Med. Chem. 2013, 70, 199- 224 and shown in Scheme 1:

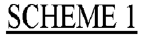

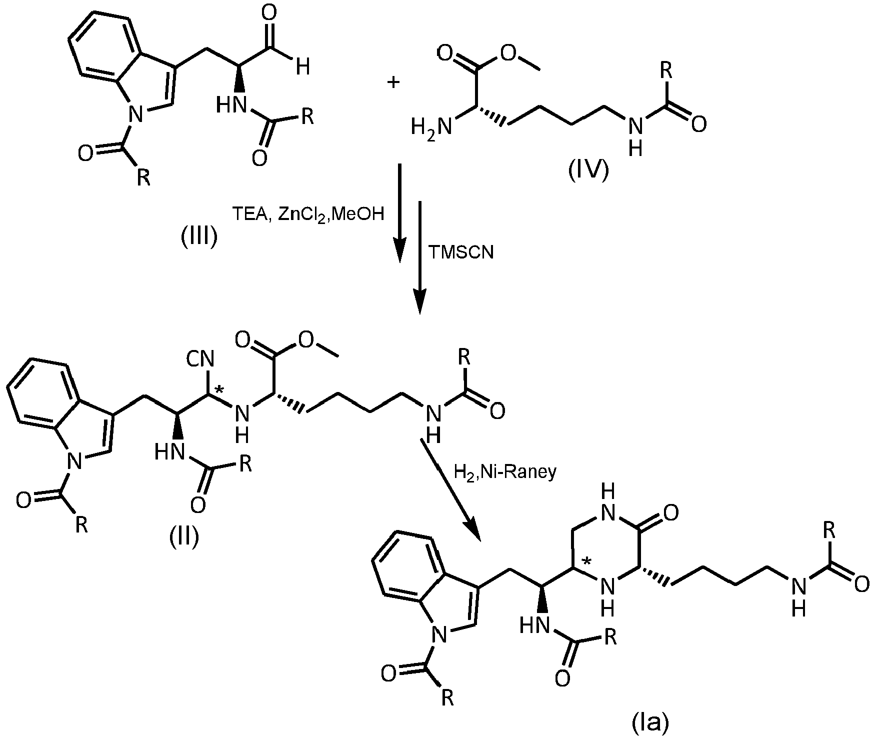

The asterisk in formulae (II) and (Ia) indicates a chiral carbon atom. The synthetic scheme depicted above yields a mixture of diastereomers which may be separated using conventional separation methods such as flash chromatography.

The compound of formula (III) may be prepared as described in WO 2012/055945 A1.

The compounds of general formula (Ib) of the present invention wherein R² is hydrogen and R¹ is different from hydrogen and R³-G¹-CO-, R⁴-G²-CO- and R⁵-G³-CO-are either *tert*-butoxycarbonyl or fluorenylmethyloxycarbonyl groups (designated as R-CO- in the following schemes) can be synthesized as described in Valdivielso, Á. M. et al. Eur. J. Med. Chem. 2013, 70, 199- 224 and shown in Scheme 2:

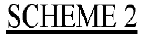

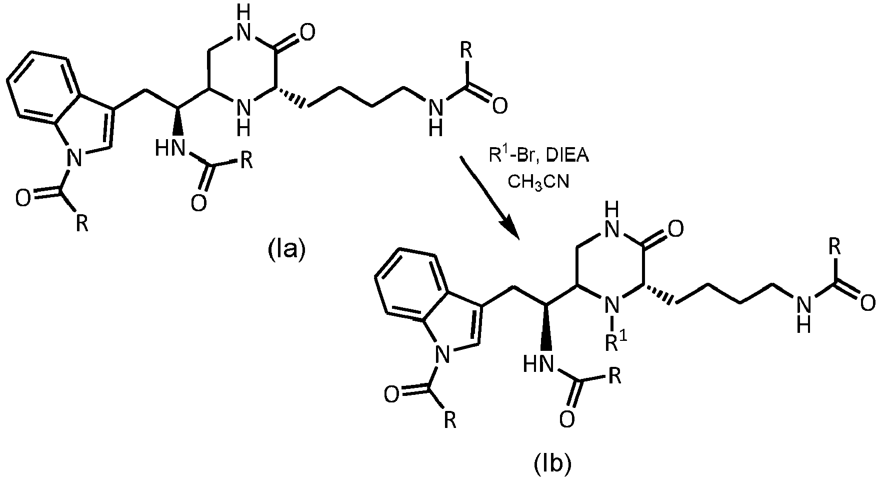

The compounds of general formula (Ic) of the present invention wherein R² is benzyl and R³-G¹-CO-, R⁴-G²-CO- and R⁵-G³-CO- are either *tert*-butoxycarbonyl or fluorenylmethyloxycarbonyl groups (designated as R-CO- in the following schemes) can be synthesized as described in Valdivielso, Á. M. et al. Eur. J. Med. Chem. 2013, 70, 199-224 and shown in Scheme 3: 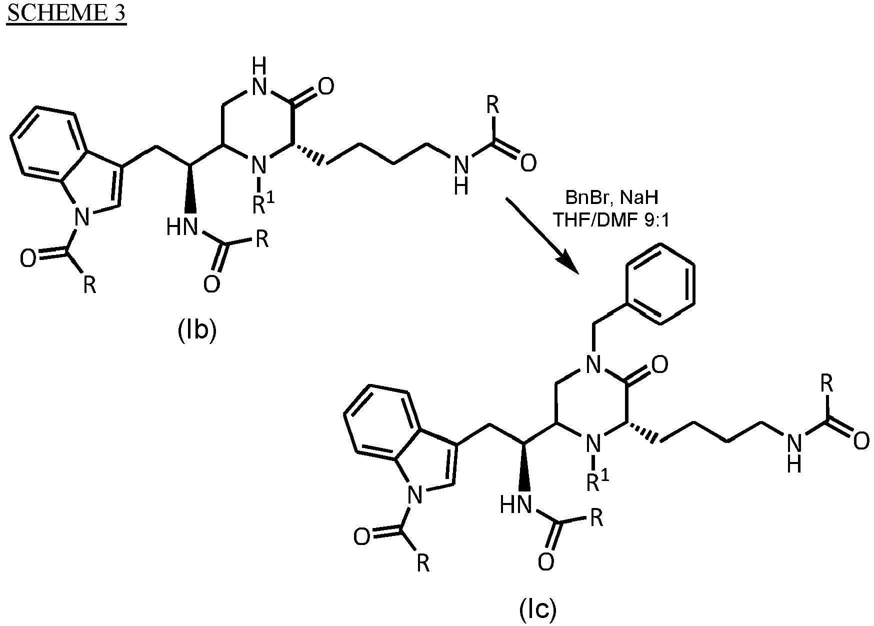 NaH (60% suspension in mineral oil, 9 mg, 0.38 mmol) and benzyl bromide (0.45 mL, 0.38 mmol) were added to a solution of the compound formula (Ib) (0.34 mmol) in anhydrous mixture THF/DMF (9:1, 10 mL) under argon at 0°C. After 1 h of stirring, the crude reaction mixture was diluted with EtOAc (100 mL) and the excess of NaH was hydrolyzed by addition of H₂O (20 mL). The aqueous layer was extracted with EtOAc (2x50 mL) and the organic extracts were dried over Na₂SO₄ and evaporated to dryness. The residue was purified by flash chromatography, using MeOH in CH₂Cl₂ gradient as mobile phase to obtain the compound of formula (Ic) in 70-80%.

The compound of formulae (Ia), (Ib) and (Ic) wherein and R³-G¹-, R⁴-G²- and R⁵-G³- are either *tert*-butoxy or fluorenylmethyloxy groups can be converted into compound where R³-G-, R⁴-G- and R⁵-G- are as defined in the claims but different than *tert*-butoxy or fluorenylmethyloxy groups following one of the following synthetic schemes 4 to 7.

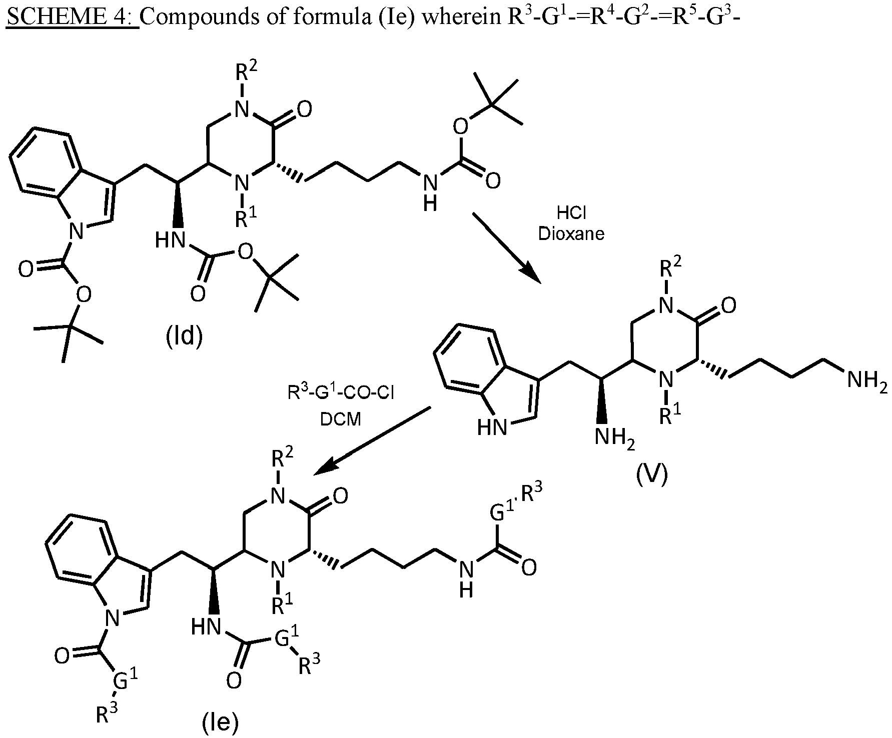

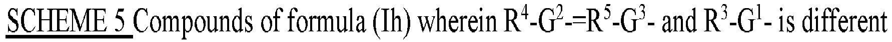

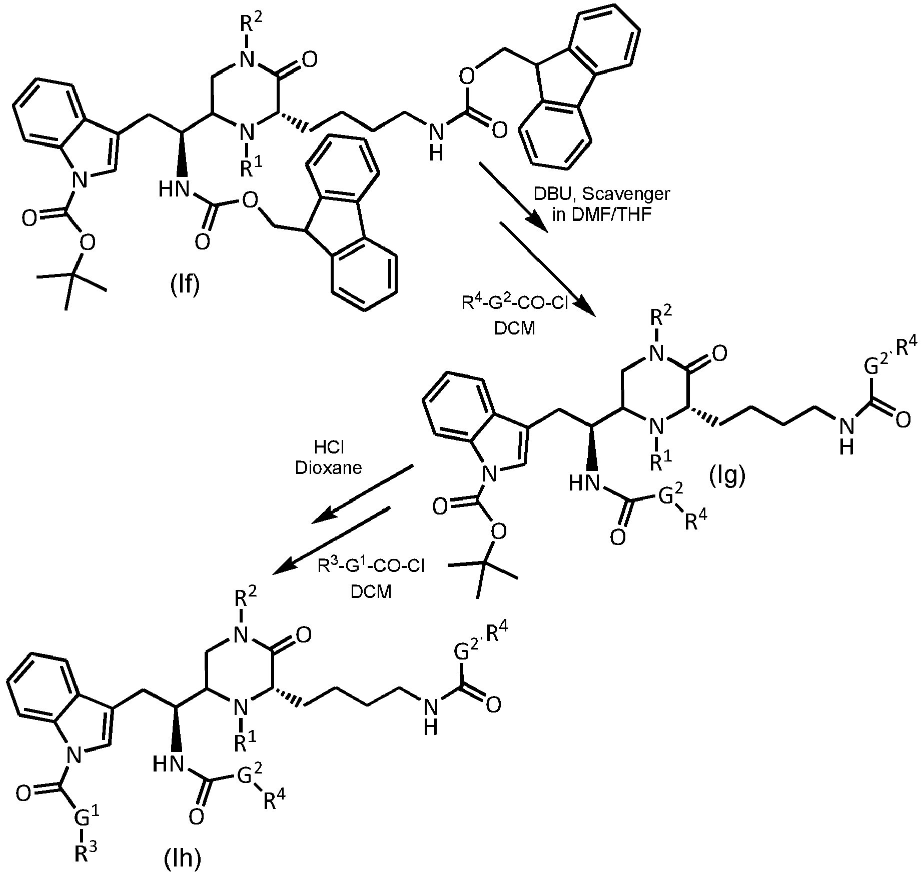

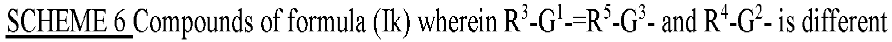

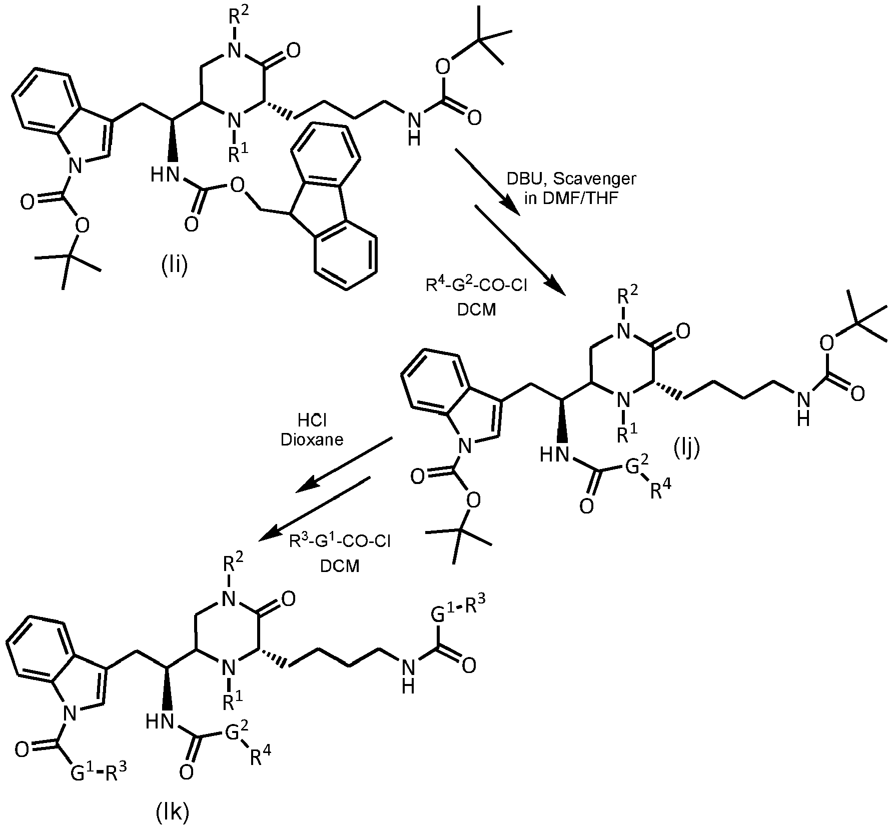

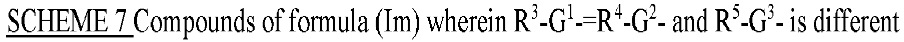

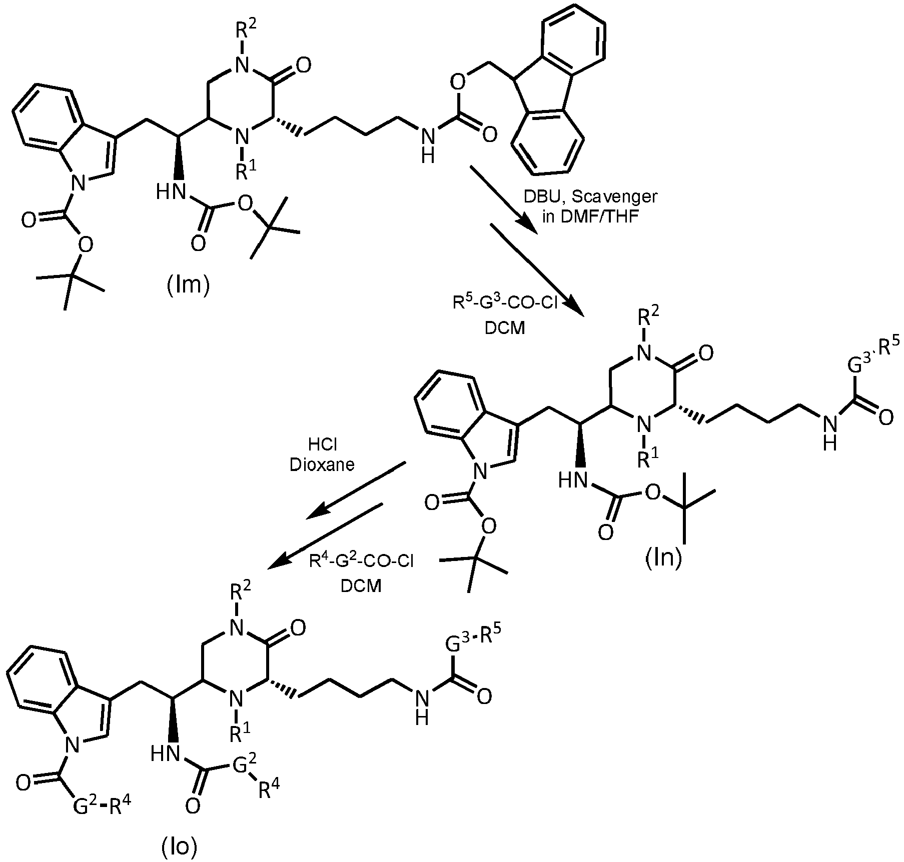

The removal of the fluorenylmethyloxycarbonyl groups (Fmoc) carried out in some of the synthetic steps of schemes 4 to 7 may be carried out following the following general method for Fmoc removal.

The corresponding Fmoc-derivative was dissolved in THF (2 mL) in a 4 mL polypropylene screw cap vial. *N*-(2-mercaptoethyl)aminomethyl polystyrene (0.5 g, 0.6 mmol/g) was added followed by 1,8-diazabicyclo[5.4.0]undec-7-ene (50 mol%) and the tube was capped under N₂ and shaken reciprocally on its side for 1.5 h or until reaction monitoring by TLC or analytical HPLC confirmed complete.

### The removal of the tert-butoxycarbonyl groups (Boc) carried out in some of the synthetic steps of schemes 4 to 7 may be carried out following the following general method for Boc removal

A solution of HCl/dioxane (4 mL, 4 M) in a 25-mL round-bottom ask equipped with a magnetic stir-bar was cooled by an ice-water bath under argon. The Boc derivative (0.2 mmol) was added in one portion with stirring. The ice-bath was removed and the mixture was kept stirred. After 30 min, TLC indicated that the reaction was completed. The reaction mixture was evaporated by rotary evaporation under high vacuum at room temperature. The residue was then washed with dry ethyl.

### The steps of acylation with groups R³-G-CO-, R⁴-G-CO- and R⁵-G-CO- may be carried out in some of the synthetic steps of schemes 4 to 7 may be carried out following the one the following general methods A, B and C.

### Method A

2.4 mmol of oxalyl chloride and a drop of DMF are added, as a catalyst, to a solution of the corresponding carboxylic acid (1 mmol) in anhydrous THF (3 mL) at 0 °C. The reaction mixture is stirred for two hours at room temperature. The acid chloride formed is dissolved in anhydrous THF (3 ml) and the corresponding amine (1.1 mmol) is added. Then, 3 equivalents of anhydrous Et₃N (3 mmol) at 0 °C are added dropwise and it is stirred overnight at room temperature. The solvent is removed under vacuum and the crude reaction product is suspended in water, acidified with IN HCl to pH=3 or 4, extracted with AcOEt and washed with a saturated solution of NaCl (3 x 15 mL). The organic phase is dried over Na₂SO₄ and concentrated under vacuum. The resulting residue is purified by medium-pressure chromatography.

### Method B

A solution of the corresponding carboxylic acid (0.75 mmol) in thionyl chloride (1.5 mL) is heated under reflux for 6 h. After this time, the excess thionyl chloride is evaporated to dryness. Then, the residue is dissolved in anhydrous THF (2 mL), and the corresponding amine (0.5 mmol) and propylene oxide (7.5 mmol) are added to the solution. The reaction is stirred at room temperature overnight. Lastly, the excess solvent is removed under vacuum and the solid formed is washed with water. The synthesized produce is purified by successive washes with the suitable solvent or by means of medium-pressure chromatography.

### Method C

2.2 mmol of DIPEA or NMM are added to a solution of the corresponding amine (0.7 mmol) in DMF (2 mL). The solution is stirred at room temperature for 10 min. Then, a coupling agent (1.1 mmol, HATU, COMU, PyAOP-HOAt, EDC, DIC, HOBt) and the corresponding acid (1.1 mmol) are added. After 12 h of stirring at room temperature, the solvent is removed at low pressure. The crude reaction product is suspended in water, acidified with 1N HCl to pH=3 or 4, extracted with AcOEt (3 x 15 mL) and washed with a saturated solution of NaCl (3 x 15 mL). The organic phase is dried over Na₂SO₄ and the solvent is evaporated to dryness. The resulting residue is purified by medium-pressure chromatography.

### Examples

HPLC-MS chromatographic data mentioned in the following examples were obtained with a Waters 2695 kit coupled to a Waters Micromass ZQ spectrometer. The separation was performed with a Sunfire C₁₈ column (3.5 µm, 4.6 mm x 50 mm), a flow of 1.0 mL/min and as a mobile phase, a gradient from A (CH₃CN) to B (H₂O) with 0.1% acid formic as an additive. Mass spectra were obtained using the ESI electrospray technique. The peaks corresponding to the molecular ion have been indicated as ([M]⁺), while those corresponding to the molecular ion plus one unit as ([M+1]⁺).

### Reference example 1 Mixture of compounds of formula (II)

TEA (1.1 mL, 7.96 mmol) was added to a solution of the compound of formula (IV) (7.96 mmol) in MeOH (40 mL). After 15 min of stirring at room temperature, the mixture was cooled to -20°C and ZnCl₂ (542 mg, 3.98 mmol) was added, followed by the addition of the compound of formula (III) (992 mg, 3.98 mmol). The solution was stirred for 1 h at -20°C, and the reaction was heated to 0°C. Then, trimethylsilyl cyanide (TMSCN) was added (896 mL, 7.16 mmol) and the mixture was stirred at 0°C during 24 h. The solvent was removed under reduced pressure and the residue was dissolved in EtOAc (50 mL).The solution was washed with H₂O (2x25 mL) and brine (25 mL), dried over Na₂SO₄, and evaporated to dryness. The residue was purified by flash chromatography, using EtOAc in hexane gradient as mobile phase to obtain the mixture of stereoisomers (II).

### (5R,3S)-5-((S)-1-(tert-butoxycarbonyl)amino-2-(1-(tert-butoxycarbonyl)(indol-3-yl))-ethyl)-3-(4-(tert-butoxycarbonyl)amino-butyl)-2-oxopiperazine and (5S,3S)-5-((S)-1-(tert-butoxycarbonyl)amino-2-(1-(tert-butoxycarbonyl)(indol-3-yl))-ethyl)-3-(4-(tert-butoxycarbonyl)namino-butyl)-2-oxopiperazine

### Examples 1 and 2 Compounds of formula (Ia)

Raney-Ni (1.50 g) was added to a solution of the corresponding epimeric mixture of compounds of formula (II) (2.88 mmol) in MeOH (15 mL) and the mixture was hydrogenated at 1 atm of H₂ at room temperature for 12 h. Afterward, the reaction mixture was filtered over celite and the solvent was evaporated under reduced pressure. The residue was purified by flash chromatography, by using MeOH in CH₂Cl₂ gradient as mobile phase to obtain the desired resolved compounds of formula (Ia): (5*R*,3*S*)-5-((*S*)-1-(*tert*-butoxycarbonyl)amino-2-(1-(*tert*-butoxycarbonyl)(indol-3-yl))-ethyl)-3-(4-(*tert-*butoxycarbonyl)amino-butyl)-2-oxopiperazine and (5*S*,3*S*)-5-((*S*)-1-(*tert-*butoxycarbonyl)amino-2-(1-(*tert*-butoxycarbonyl)(indol-3-yl))-ethyl)-3-(4-(*tert-*butoxycarbonyl)amino-butyl)-2-oxopiperazine

### Example 1: (Compound (Ia) (R)-isomer) (5R,3S)-5-((S)-1-(tert-butoxycarbonyl)amino-2-(1-(tert-butoxycarbonyl)(indol-3-yl))-ethyl)-3-(4-(tert-butoxycarbonyl)amino-butyl)-2-oxopiperazine

White solid (726 mg, 40%); [α]_{D}²⁰ -12 (*c* 1.6, CH₂Cl₂); mp: 84-86°C (EtOAc/hexane); HPLC [Sunfire C₁₈ (4.6x150 mm, 3.5 µm), 30-100% gradient of solvent A in B, 30 min] t_{R} 13.89 min;
¹H NMR ((400 MHz, (CD₃)₂CO) δ (ppm): 1.31 (s, 9H, Boc), 1.37 [s, 9H, Boc(Lys)], 1.52 [m, 2H, γ-H (Lys)], 1.54 [m, 2H, δ-H (Lys)],1.66 [s, 9H, Boc(Ind)], 1.69 [m, 1H, β-H(Lys)], 1.84 [m, 1H, β-H (Lys)], 2.40 (m, 1H, 4-H), 2.89 (m, 1H, CH₂-Ind), 3.10 [m, 2H, ε-H (Lys)], 3.17 (m, 1H, 5-H), 3.26 (m, 1H, 6-H), 3.31 (m, 1H, 3-H), 3.43 (m, 1H, CH₂-Ind), 3.47 (m, 1H, 6-H), 3.87 (ddd, 1H, J = 3, 9.5 and 18.5 Hz, 5-CH), 5.95 [m, 1H, NHBoc(Lys)], 6.01 (d, 1H, J = 9.5 Hz, NHBoc), 6.78 (s, 1H, 1-H), 7.23 (t, 1H, J = 7.5 Hz, Ind), 7.30 (t, 1H, J =7.5 Hz, Ind), 7.51 (m, 1H, Ind), 7.63 (d, 1H, J = 7.5 Hz, Ind), 8.12 (d, 1H, J = 7.5 Hz, Ind).
¹³C NMR (100 MHz, (CD₃)₂CO) δ (ppm): 24.6 [C_{γ} (Lys)], 27.7 [CH₂-Ind], 28.3, 28.6, 28.7 [9CH₃ (3Boc)], 30.7 [C_{δ} (Lys)], 31.2 [C_{β} (Lys)], 41.2 [C_{ε}(Lys)], 46.4 (C₆), 52.0 (C₅), 53.6 (Cs-CH), 57.7 (C₃), 78.3, 78.7, 83.9 [3C(3Boc)], 115.8 [CH(Ind)], 118.9 [C(Ind)], 120.1, 123.1, 124.3, 124.9 [4CH (Ind)], 132.1, 136.3 [2C (Ind)], 150.3, 156.6 [3CO (3Boc)], 173.0 (C₂); ES-MS m/z 630.8 [M+1]⁺; Anal. calcd. for C₃₃H₅₁N₅O₇: C, 62.93; H, 8.16; N, 11.12. Found: C, 62.66; H, 8.37; N, 10.95.

### Example 2: (Compound (Ia) (S)-isomer): (5S,3S)-5-((S)-1-(tert-butoxycarbonyl)amino-2-(1-(tert-butoxycarbonyl)(indol-3-yl))-ethyl)-3-(4-(tert-butoxycarbonyl)amino-butyl)-2-oxopiperazine

White solid (363 mg, 20%); [α]_{D}²⁰ -10 (*c* 1.4, CH₂Cl₂); mp: 76-78 °C (EtOAc/hexane); HPLC [Sunfire C₁₈ (4.6 x 150 mm, 3.5 µm), 30-100% gradient of solvent A in 30 min] *t*_{R} 13.56 min;
¹H NMR (400 MHz, (CD₃)₂CO) δ (ppm): 1.35 (s, 9H, Boc), 1.39 [s, 9H, Boc(Lys)], 1.48 [m, 2H, γ-H(Lys)], 1.50 [m, 2H, δ-H(Lys)], 1.59 [m, 1H, β-H(Lys)], 1.67 [s, 9H, Boc (Ind)], 1.94 [m, 1H, β-H(Lys)], 2.99 (dd, 1H, J = 8 and 14.5 Hz, CH₂-Ind), 3.08 [m, 2H, ε-H(Lys)], 3.10 (m, 1H, CH₂-Ind), 3.12 (m, 1H, 5-H), 3.26 (m, 2H, 6-H), 3.27 (m, 1H, 3-H), 4.00 (m, 1H, 5-CH), 5.94 [m, 1H, NHBoc (Lys)], 6.06 (d, 1H, J = 9 Hz, NHBoc), 6.73 (s, 1H, 1-H), 7.25 (t, 1H, J = 7.5 Hz, Ind), 7.31 (t, 1H, J = 7.5 Hz, Ind), 7.55 (m, 1H, Ind), 7.70 (d, 1H, J = 7.5 Hz, Ind), 8.13 (d, 1H, J = 7.5 Hz, Ind).
¹³C NMR (100 MHz, (CD₃)₂CO) δ (ppm): 23.9 [C_{γ}(Lys)], 28.0 [CH₂-Ind], 28.3, 28.6, 28.8 [9CH₃ (3Boc)], 31.0 [C_{δ} (Lys)], 32.6 [C_{β} (Lys)], 41.1 [C_{ε} (Lys)], 46.2 (C₆), 53.0 (C₅-CH), 55.5 (C₅), 59.7 (C₃), 78.3, 78.9, 84.0 [3C (3Boc)], 115.6 [CH (Ind)], 118.5 [C (Ind)], 120.1, 123.3, 124.6, 125.0 [4CH (Ind)], 131.8, 136.4 [2C (Ind)], 150.3, 156.7 [3CO (3Boc)], 171.7 (C₂);
ES-MS m/z 630.8 [M+1]⁺;
Anal. calcd. for C₃₃H₅₁N₅O7: C, 62.93; H, 8.16; N, 11.12. Found: C, 62.83; H, 8.02; N, 10.90.

### Examples 3 and 4 Compounds of formula (Ib) wherein R¹ is benzyl

DIEA (456 mL, 2.71 mmol) and benzyl bromide (541 µL, 2.71 mmol) were added under argon in four equal portions over 72 h to a solution of either the compounds of example 1 (to obtain the compound of example 3) or 2 (to obtain the compound of example 4) (0.66 mmol) in anhydrous CH₃CN (10 mL) at 60 °C. After 4 days of stirring, the solvent was removed under reduced pressure and the residue was dissolved in CH₂Cl₂ (100 mL). The solution was successively washed with H₂O (2 x 25 mL) and brine (25 mL), dried over Na₂SO₄ and evaporated to dryness. The residue was purified by flash chromatography, using MeOH in CH₂Cl₂ gradient as mobile phase to obtain the respective compounds.

### Example 3: (Compound (Ib) (R)-isomer) (5R,3S)-4-benzyl-5-((S)-1-(tert-butoxycarbonyl)amino-2-(1-(tert-butoxycarbonyl)(indol-3-yl))-ethyl)-3-(4-(tert-butoxycarbonyl)amino-butyl)-2-oxopiperazine

Reddish solid (333 mg, 70%); [α]_{D}²⁰ +0.9 (*c* 1.1, CH₂Cl₂);
mp: 76-78 °C (EtOAc/hexane);
HPLC [Sunfire C₁₈ (4.6 x 150 mm, 3.5 µm), 70-100% gradient of solvent A in B, 30 min] *t*_{R} 11.27 min;
¹H NMR (400 MHz, (CD₃)₂CO) δ (ppm): 1.29 [m, 2H, γ-H (Lys)], 1.30 (s, 9H, Boc), 1.34 [m, 2H, δ-H (Lys)], 1.36 [s, 9H, Boc (Lys)], 1.61 [m, 1H, β-H (Lys)], 1.66 [s, 9H, Boc (Ind)], 1.72 [m, 1H, β-H (Lys)], 1.83 [m, 1H, ε-H (Lys)], 2.88 (dd, 1H, J = 11 and 15 Hz, CH₂-Ind), 3.00 [m, 1H, ε-H (Lys)], 3.04 (m, 1H, 3-H), 3.49 (m, 1H, 5-H), 3.50 (m, 1H, 6-H), 3.60 [m, 2H, 4-CH₂ (Bn) and 6-H], 3.63 (m, 1H, CH₂-Ind), 4.22 [m, 1H, 4-CH₂ (Bn)], 4.23 (m, 1H, 5-CH), 5.80 [m, 1H, NHBoc (Lys)], 6.08 (d, 1H, J = 9.5 Hz, NHBoc), 6.82 (s, 1H, 1-H), 7.18 (t, 1H, J = 7.5 Hz, Ar), 7.29 (m, 2H, Ar), 7.52 (m, 2H, Ar), 7.53 (m, 4H, Ar), 8.12 (d, 1H, J = 7.5 Hz, Ar).
¹³C NMR (100 MHz, (CD₃)₂CO) δ (ppm): 24.5 [C_{γ} (Lys)], 28.2 [CH₂-Ind], 28.3, 28.5, 28.7 [9CH₃ (3Boc)], 30.3 [C_{δ} (Lys)], 31.1 [C_{β} (Lys)], 41.1 [C_{ε} (Lys)], 41.5 (C₆), 51.3 [C₅-CH], 51.9 [4-CH₂ (Bn)], 55.4 (C₅), 62.2 (C₃), 78.8, 83.9 [3C (3Boc)], 115.8 [CH (Ar)], 118.8 [C (Ar)], 119.9, 123.2, 124.3, 124.9, 128.0, 129.3, 129.9 [9CH (Ar)], 132.0, 136.4, 139.7 [3C (Ar)], 150.3, 156.6 [3CO (3Boc)], 172.0 (C₂);
ES-MS m/z 720.9 [M+1]⁺;
Anal. calcd. for C₄₀H₅₇N₅O₇: C, 66.73; H, 7.98; N, 9.73. Found: C, 66.51; H, 8.15; N, 9.95.

### Example 4: (Compound (Ib) (S)-isomer) (5R,3S)-4-benzyl-5-((S)-1-(tert-butoxycarbonyl)amino-2-(1-(tert-butoxycarbonyl)(indol-3-yl))-ethyl)-3-(4-(tert-butoxycarbonyl)amino-butyl)-2-oxopiperazine

Reddish solid (381 mg, 80%); [α]_{D}²⁰ +13.6 (c 1.2, CH₂Cl₂);
mp: 84-86 °C (EtOAc/hexane);
HPLC [Sunfire C₁₈ (4.6 x150 mm, 3.5 µm), 70-100% gradient of solvent A in B 30 min] *t*_{R} 11.86 min;
¹H NMR (400 MHz, (CD₃)₂CO) δ (ppm): 1.21 [m, 2H, γ-H (Lys)], 1.31 [m, 2H, δ-H (Lys)],1.36 [m,19H, Boc, Boc (Lys) and β-H (Lys)],1.67 [s, 9H, Boc (Ind)], 1.68 [m, 1H, β-H (Lys)], 2.92 (m, 1H, CH₂-Ind), 2.94 [m, 2H, ε-H (Lys)], 3.07 (t, 1H, J = 7 Hz, 3-H), 3.14 (m, 1H, 5-H), 3.24 (dd, 1H, J = 2.5 and 15 Hz, CH₂-Ind), 3.53 (m, 2H, 6-H), 3.80 [d, 1H, J = 14 Hz, 4-CH₂ (Bn)], 4.08 [d, 1H, J = 14 Hz, 4-CH₂ (Bn)], 4.17 (m, 1H, 5-CH), 5.82 [m, 1H, NHBoc (Lys)], 6.13 (d, 1H, J = 8.5 Hz, NHBoc), 7.03 (s, 1H, 1-H), 7.26 (m, 2H, Ar), 7.34 (m, 3H, Ar), 7.51 (m, 3H, Ar), 7.66 (d, 1H, J = 7.5 Hz, Ar), 8.14 (d, 1H, J = 7.5 Hz, Ar).
¹³C NMR (100 MHz, (CD₃)₂CO) δ (ppm): 24.1 [C_{γ} (Lys)], 26.6 [CH₂-Ind], 28.3, 28.7 [9CH₃ (3Boc)], 30.0 [C_{δ} (Lys)], 34.4 [C_{β} (Lys)], 40.2 (C₆), 40.9 [C_{ε} (Lys)], 52.9 [Cs-CH], 62.4 [4-CH₂ (Bn)], 63.9 (C₅), 64.0 (C₃), 78.2, 78.8, 84.0 [3C (3Boc)], 115.9 [CH (Ar)], 119.0 [C (Ar)], 120.0, 123.2, 124.0, 125.0, 128.0, 129.1, 130.2 [9CH (Ar)], 131.9, 136.4, 140.2 [3C (Ar)], 150.3, 156.6 [3CO (3Boc)], 173.2 (C₂);
ES-MS m/z 720.9 [M+1]⁺;
Anal. calcd. for C₄₀H₅₇N₅O₇: C, 66.73; H, 7.98; N, 9.73. Found: C, 66.49; H, 8.21; N, 9.51.

### Examples 5 and 6 Compounds of formula (Ib) wherein R¹ is tert-butoxycarbonylmethyl

DIEA (456 mL, 2.71 mmol) and tert-butyl bromoacetate (540 µL, 2.71 mmol) were added under argon in four equal portions over 72 h to a solution of either the compounds of example 1 (to obtain the compound of example 5) or 2 (to obtain the compound of example 6) (0.66 mmol) in anhydrous CH₃CN (10 mL) at 60 °C. After 4 days of stirring, the solvent was removed under reduced pressure and the residue was dissolved in CH₂Cl₂ (100 mL). The solution was successively washed with H₂O (2 x 25 mL) and brine (25 mL), dried over Na₂SO₄ and evaporated to dryness. The residue was purified by flash chromatography, using MeOH in CH₂Cl₂ gradient as mobile phase to obtain the respective compounds.

### Example 5: (5R,3S)-5-((S)-1-(tert-butoxycarbonyl)amino-2-(1-(tert-butoxycarbonyl)-(indol-3-yl))-ethyl)-4(tert-butoxycarbonyl)methyl-3-(4-(tert-butoxycarbonyl)aminobutyl)-2-oxopiperazine

Reddish solid (368 mg, 75%);
[α]_{D}²⁰ +3 (c 1.7, CH₂Cl₂);
mp: 78-80 °C (EtOAc/hexane);
HPLC [Sunfire C₁₈ (4.6 x 150 mm, 3.5 µm), 70-100% gradient of solvent A in B, 30 min] *t*_{R} 11.54 min;
¹H NMR (400 MHz, (CD₃)₂CO) δ (ppm): 1.29 (s, 9H, Boc), 1.36 [s, 9H, Boc(Lys)], 1.49 (s, 9H, ^{t}Bu), 1.59 [m, 2H, γ-H (Lys)], 1.60 [m, 2H, δ-H (Lys)],1.67 [s, 9H, Boc(Ind)], 1.79 [m, 1H, β-H (Lys)], 1.80 [m, 1H, β-H (Lys)], 2.78 (dd, 1H, J = 11 and 15 Hz, CH₂-Ind), 3.12 [m, 2H, ε-H (Lys)], 3.19 (m, 2H, 3-H and 4-CH₂), 3.39 (m, 1H, 5-H), 3.41 (m, 2H, 6-H), 3.64 (d, 1H, J = 16 Hz, 4-CH2), 3.66 (dd, 1H, J = 2 and 15 Hz, CH₂-Ind), 3.94 (m, 1H, 5-CH), 5.93 [m, 1H, NHBoc (Lys)], 6.07 (d, 1H, J = 9.5 Hz, NHBoc), 6.84 (s, 1H, 1-H), 7.23 (t, 1H, J = 7.5 Hz, Ind), 7.30 (t, 1H, J = 7.5 Hz, Ind), 7.51 (s, 1H, Ind), 7.64 (d, 1H, J = 7.5 Hz, Ind), 8.13 (d, 1H, J = 7.5 Hz, Ind).
¹³C NMR (100 MHz, (CD₃)₂CO) δ (ppm): 24.7 [C_{γ} (Lys)], 27.8 (CH₂-Ind), 28.3 [6CH₃ (Boc and ^{t}Bu)], 28.5, 28.7 [6CH₃ (2Boc)], 30.4 [C_{δ} (Lys)], 31.1 [C_{β} (Lys)], 41.2 [C_{ε} (Lys)], 41.8 (C₆), 51.9 (C₅-CH), 52.1 (4-CH₂), 55.8 (C₅), 65.2 (C₃), 78.2, 78.8 [2C (2Boc)], 81.5 [C (^{t}Bu)], 83.9 [C (Boc)], 115.8 [CH (Ind)], 118.9 [C (Ind)], 120.0, 123.1, 124.3, 124.9 [4CH (Ind)], 132.0, 136.4 [2C (Ind)], 150.3, 156.6 [3CO (3Boc)], 171.0 (CO₂), 171.8 (C₂);
ES-MS m/z 744.9 [M+1]⁺;
Anal. calcd. for C₃₉H₆₁N₅O₉: C, 62.97; H, 8.26; N, 9.41. Found: C, 63.21; H, 8.15; N, 9.24.

### Example 6: (5S,3S)-5-((S)-1-(tert-butoxycarbonyl)amino-2-(1-(tert-butoxycarbonyl)-(indol-3-yl))-ethyl)-4(tert-butoxycarbonyl)methyl-3-(4-(tert-butoxycarbonyl)aminobutyl)-2-oxopiperazine

Reddish solid (295 mg, 60%);
[α]_{D}²⁰ +10 (*c* 1.2, CH₂Cl₂);
mp: 80-82 °C (EtOAc/hexane);
HPLC [Sunfire C₁₈ (4.6 x 150 mm, 3.5 µm), 70-100% gradient of solvent A in B, 30 min] *t*_{R} 12.04 min;
¹H NMR (400 MHz, (CD₃)₂CO) δ (ppm): 1.34 (s, 9H, Boc), 1.37 [s, 9H, Boc (Lys)], 1.45 (s, 9H, ^{t}Bu), 1.53 [m, 2H, γ-H (Lys)], 1.56 [m, 2H, δ-H (Lys)], 1.66 [s, 9H, Boc (Ind)], 1.77 [m, 2H, β-H (Lys)], 2.80 (m, 1H, CH₂-Ind), 3.07 [m, 2H, ε-H (Lys)], 3.23 (m, 1H, CH₂-Ind), 3.27 (m, 1H, 5-H), 3.30 (m, 1H, 3-H), 3.50 (m, 2H, 6-H), 3.57 (d, 1H, J = 18 Hz, 4-CH₂), 3.68 (d, 1H, J = 18 Hz, 4-CH₂), 4.07 (m, 1H, 5-CH), 5.93 [m, 1H, NHBoc (Lys)], 6.21 (d, 1H, J = 8.5 Hz, NHBoc), 7.00 (s, 1H, 1-H), 7.26 (t, 1H, J = 7.5 Hz, Ind), 7.31 (t, 1H, J = 7.5 Hz, Ind), 7.52 (s, 1H, Ind), 7.67 (d, 1H, J = 7.5 Hz, Ind), 8.13 (d, 1H, J = 7.5 Hz, Ind).
¹³C NMR (100 MHz, (CD₃)₂CO) δ (ppm): 23.8 [C_{γ} (Lys)], 25.6 (CH₂-Ind), 28.3 [6CH₃ (Boc and ^{t}Bu)], 28.6 [6CH₃ (2Boc)], 30.4 [C_{δ} (Lys)], 34.5 [C_{β} (Lys)], 39.9 (C₆), 41.1 [C_{ε} (Lys)], 52.0 (C₅-CH), 57.0 (4-CH₂), 60.1 (C₅), 65.3 (C₃), 78.2, 78.8 [2C (2Boc)], 81.7 [C (^{t}Bu)], 84.0 [C (Boc)], 115.9 [CH (Ind)],119.1 [C (Ind)],119.6,123.3,124.1,125.0 [4CH (Ind)],131.8, 136.4 [2C (Ind)], 150.3, 156.4 [3CO (3Boc)], 171.3 (CO₂), 172.6 (C₂);
ES-MS m/z 744.9 [M+1]⁺;
Anal. calcd. for C₃₉H₆₁N₅O₉: C, 62.97; H, 8.26; N, 9.41. Found: C, 62.71; H, 8.05; N, 9.63.

### Examples 7 and 8 Compounds of formula (Ic) wherein R¹ is benzyl

NaH (60% suspension in mineral oil, 9 mg, 0.38 mmol) and benzyl bromide (0.45 mL, 0.38 mmol) were added to a solution of either the compounds of example 3 (to obtain the compound of example 7) or 4 (to obtain the compound of example 8) (0.34 mmol) in anhydrous mixture THF/DMF (9:1, 10 mL) under argon at 0 °C. After 1 h of stirring, the crude reaction mixture was diluted with EtOAc (100 mL) and the excess of NaH was hydrolyzed by addition of H₂O (20 mL). The aqueous layer was extracted with EtOAc (2 x 50 mL) and the organic extracts were dried over Na₂SO₄ and evaporated to dryness. The residue was purified by flash chromatography, using MeOH in CH₂Cl₂ gradient as mobile phase to obtain the respective compounds in 70-80%.

### Example 7: (5R,3S)-1,4-dibenzyl-5-((S)-1-(tert-butoxycarbonyl)amino-2-(1-(tert-butoxycarbonyl)(indol-3-yl))-ethyl)-3-(4-(tert-butoxycarbonyl)amino-butyl)-2-oxopiperazine

Amorphous solid (220 mg, 80%);
[α]_{D}²⁰ -15 (c 1.4, CH₂Cl₂);
HPLC [Sunfire C₁₈ (4.6 x 150 mm, 3.5 µm), 80-100% gradient of solvent A in B, 30 min] *t*_{R} 12.75 min;
¹H NMR (400 MHz, (CD₃)₂CO) δ (ppm): 1.25 (s, 9H, Boc), 1.31 [m, 4H, γ-H (Lys) and δ-H (Lys)], 1.36 [s, 9H, Boc (Lys)], 1.61 [m, 1H, β-H (Lys)], 1.64 [s, 9H, Boc (Ind)], 1.80 [m, 1H, β-H (Lys)], 1.86 [m, 1H, ε-H (Lys)], 2.80 (m, 1H, CH₂-Ind), 3.03 [m, 1H, ε-H (Lys)], 3.17 (dd, 1H, J = 4.5 and 10 Hz, 3-H), 3.37 (m, 1H, 6-H), 3.54 [m, 1H, 4-CH₂ (Bn)], 3.59 (m, 3H, 5-H, 6-H and CH₂-Ind), 4.17 (m, 1H, 5-CH), 4.18 [d, 1H, J = 13.5 Hz, 4-CH₂ (Bn)], 4.51 [d, 1H, J = 14.5 Hz, 1-CH₂ (Bn)], 4.79 [d, 1H, J = 14.5 Hz, 1-CH₂ (Bn)], 5.84 [m, 1H, NHBoc (Lys)], 6.00 (d, 1H, J = 9.5 Hz, NHBoc), 7.16 (t, 1H, J = 7.5 Hz, Ar), 7.22-7.57 (m, 13H, Ar), 8.12 (d, 1H, J = 7.5 Hz, Ar);
¹³C NMR (100 MHz, (CD₃)₂CO) δ (ppm): 24.5 [C_{γ} (Lys)], 27.9 (CH₂-Ind), 28.3, 28.5, 28.7 [9CH₃ (3Boc)], 30.3 [C_{δ} (Lys)], 31.2 [C_{β} (Lys)], 41.1 [C_{ε} (Lys)], 45.8 (C₆), 50.2 [1-CH₂ (Bn)], 51.3 (Cs-CH), 51.9 [4-CH₂ (Bn)], 55.6 (C₅), 62.5 (C₃), 78.2, 78.9, 83.9 [3C (3Boc)],115.8 [CH (Ar)],118.7 [C (Ar)], 119.9, 123.2, 124.3, 124.9, 128.1, 128.2, 128.9, 129.3, 129.4, 129.9 [14CH (Ar)], 131.9, 136.3, 138.9, 139.5 [4C (Ar)], 150.3, 156.5 [3CO (3Boc)], 171.0 (C₂);
ES-MS m/z 811.2 [M+1]⁺;
Anal. calcd. For C₄₇H₆₃N₅O₇: C, 69.69; H, 7.84; N, 8.65. Found: C, 69.92; H, 8.06; N, 8.41.

### Example 8: (5S,3S)-1,4-dibenzyl-5-((S)-1-(tert-butoxycarbonyl)amino-2-(1-(tert-butoxycarbonyl)(indol-3-yl))-ethyl)-3-(4-(tert-butoxycarbonyl)amino-butyl)-2-oxopiperazine

Amorphous solid (220 mg, 80%);
[α]_{D}²⁰ +11 (c 1.3, CH₂Cl₂);
HPLC [Sunfire C₁₈ (4.6 x 150 mm, 3.5 µm), 80-100% gradient of solvent A in B, 30 min] *t*_{R} 12.99 min;
¹H NMR (400 MHz, (CD₃)₂CO) δ (ppm): 1.28 [m, 2H, γ-H (Lys)], 1.31 [m, 9H, Boc],1.34 [m, 2H, δ-H (Lys)],1.37 [m, 9H, Boc (Lys)], 1.43 [m, 1H, β-H (Lys)], 1.66 [s, 9H, Boc (Ind)], 1.71 [m, 1H, β-H (Lys)], 2.85 (m, 1H, CH₂-Ind), 2.95 [m, 2H, ε-H (Lys)], 3.16 (m, 1H, 5-H), 3.19 (m, 1H, CH₂-Ind), 3.27 (t, 1H, J = 7 Hz, 3-H), 3.46 (dd, 1H, J = 5 and 13.5 Hz, 6-H), 3.57 (m, 1H, 6-H), 3.85 [d, 1H, J = 14 Hz, 4-CH₂ (Bn)], 4.08 [d, 1H, J = 14 Hz, 4-CH₂ (Bn)], 4.12 (m, 1H, 5-CH), 4.57 [d, 1H, J = 15 Hz, 1-CH₂ (Bn)], 4.66 [d, 1H, J = 15 Hz, 1-CH₂ (Bn)], 5.80 [m, 1H, NHBoc (Lys)], 6.09 (d, 1H, J = 9 Hz, NHBoc), 7.18-7.38 (m, 10H, Ar), 7.47 (m, 3H, Ar), 7.61 (d, 1H, J = 7.5 Hz, Ar), 8.12 (d, 1H, J = 7.5 Hz, Ar).
¹³C NMR (100 MHz, (CD₃)₂CO) δ (ppm): 24.2 [C_{γ} (Lys)], 26.4 (CH₂-Ind), 28.3, 28.7 [9CH₃ (3Boc)], 31.2 [C_{δ} (Lys)], 34.8 [C_{β} (Lys)], 41.0 [C_{ε} (Lys)], 45.0 [C₆], 49.8 [1-CH₂ (Bn)], 52.8 (Cs-CH), 62.3 [4-CH₂ (Bn)], 63.6 (C₅), 64.3 (C₃), 78.2, 78.8, 84.0 [3C (3Boc)], 115.9 [CH (Ar)], 119.0 [C (Ar)], 119.9, 123.2, 124.0, 125.0, 128.1, 128.7, 129.1, 129.4, 130.2 [14CH (Ar)], 131.8, 136.3, 138.7, 139.9 [4C (Ar)], 150.3, 156.5 [3CO (3Boc)], 171.7 (C₂);
ES-MS m/z 811.2 [M+1]⁺;
Anal. calcd. for C₄₇H₆₃N₅O₇: C, 69.69; H, 7.84; N, 8.65. Found: C, 69.90; H, 7.96; N, 8.52.

### Examples 9 and 10 Compounds of formula (Ic) wherein R¹ is tert-butoxycarbonylmethyl

NaH (60% suspension in mineral oil, 9 mg, 0.38 mmol) and benzyl bromide (0.45 mL, 0.38 mmol) were added to a solution of either the compounds of example 5 (to obtain the compound of example 9) or 6 (to obtain the compound of example 10) (0.34 mmol) in anhydrous mixture THF/DMF (9:1, 10 mL) under argon at 0 °C. After 1 h of stirring, the crude reaction mixture was diluted with EtOAc (100 mL) and the excess of NaH was hydrolyzed by addition of H₂O (20 mL). The aqueous layer was extracted with EtOAc (2 x 50 mL) and the organic extracts were dried over Na₂SO₄ and evaporated to dryness. The residue was purified by flash chromatography, using MeOH in CH₂Cl₂ gradient as mobile phase to obtain the respective compounds in 70-80%.

### Example 9: (5R,3S)-1-benzyl-5-((S)-1-(tert-butoxycarbonyl)amino-2-(1-(tert-butoxycarbonyl)(indol-3-yl))-ethyl)-4-(tert-butoxycarbonyl)methyl-3-(4-(tert-butoxycarbonyl)amino-butyl)-2-oxopiperazine

Amorphous solid (227 mg, 80%);
[α]_{D}²⁰ -4.2 (*c* 1.5, CH₂Cl₂);
HPLC [Sunfire C₁₈ (4.6 x 150 mm, 3.5 µm), 80-100% gradient of solvent A in B, 30 min] *t*_{R} 11.80 min;
¹H NMR (400 MHz, (CD₃)₂CO) δ (ppm): 1.23 (s, 9H, Boc), 1.37 [s, 9H, Boc (Lys)], 1.48 (s, 9H, ^{t}Bu), 1.61 [m, 2H, γ-H (Lys)], 1.63 [m, 2H, δ-H (Lys)], 1.65 [s, 9H, Boc (Ind)], 1.79 [m, 1H, β-H (Lys)], 1.87 [m, 1H, β-H (Lys)], 2.75 (dd, 1H, J = 10.5 and 15 Hz, CH₂-Ind), 3.13 (m, 1H, 4-CH₂), 3.16 [m, 2H, ε-H (Lys)], 3.29 (m, 1H, 6-H), 3.33 (m, 1H, 3-H), 3.40 (m, 1H, 6-H), 3.46 (m, 1H, 5-H), 3.61 (m, 1H, CH₂-Ind), 3.62 (d, 1H, J = 16 Hz, 4-CH₂), 3.91 (m, 1H, 5-CH), 4.38 [d, 1H, J = 14.5 Hz, 1-CH₂ (Bn)], 4.84 [d, 1H, J = 14.5 Hz, 1-CH₂ (Bn)], 5.93 [m, 1H, NHBoc (Lys)], 5.98 (d, 1H, J = 9.5 Hz, NHBoc), 7.22 (t, 1H, J = 7.5 Hz, Ar), 7.25-7.37 (m, 6H, Ar), 7.47 (s, 1H, Ind), 7.62 (d, 1H, J = 7.5 Hz, Ind), 8.11 (d, 1H, J = 7.5 Hz, Ind);
¹³C NMR (100 MHz, (CD₃)₂CO) δ (ppm): 24.7 [C_{γ} (Lys)], 27.6 (CH₂-Ind), 28.3 [6CH₃ (Boc and ^{t}Bu)], 28.5, 28.7 [6CH₃ (2Boc)], 30.4 [C_{δ} (Lys)], 31.3 [C_{β} (Lys)], 41.2 [C_{ε} (Lys)], 46.0 (C₆), 50.2 [1-CH₂ (Bn)], 51.9 (Cs-CH), 52.1 (4-CH₂), 56.0 (C₅), 65.6 (C₃), 78.2, 78.9 [2C (2Boc)], 81.5 [C (^{t}Bu)], 83.9 [C (Boc)], 115.8 [CH (Ar)], 118.9 [C (Ar)], 120.0, 123.1, 124.4, 124.9, 128.1, 128.8, 129.4 [9CH (Ar)], 131.9, 136.3, 138.7 [3C (Ar)],150.3, 156.4 [3CO (3Boc)], 170.7 [CO₂], 170.8 (C₂);
ES-MS m/z 835.1 [M+1]⁺;
Anal. calcd. for C₄₆H₆₇N₅O₉: C, 66.24; H, 8.10; N, 8.40. Found: C, 66.03; H, 7.86; N, 8.35.

### Example 10: (5S,3S)-1-benzyl-5-((S)-1-(tert-butoxycarbonyl)amino-2-(1-(tert-butoxycarbonyl)(indol-3-yl))-ethyl)-4-(tert-butoxycarbonyl)methyl-3-(4-(tert-butoxycarbonyl)amino-butyl)-2-oxopiperazine

Amorphous solid (198 mg, 70%);
[α]_{D}²⁰ +7.0 (c 1.3, CH₂Cl₂);
HPLC [Sunfire C₁₈ (4.6 x 150 mm, 3.5 µm), 80-100% gradient of solvent A in B, 30 min] *t*_{R} 12.41 min;
¹H NMR (400 MHz, (CD₃)₂CO) δ (ppm): 1.29 (s, 9H, Boc), 1.37 [s, 9H, Boc (Lys)], 1.45 (s, 9H, ^{t}Bu), 1.55 [m, 2H, γ-H (Lys)], 1.57 [m, 2H, δ-H (Lys)], 1.65 [s, 9H, Boc (Ind)], 1.80 [m, 2H, β-H (Lys)], 2.71 (dd, 1H, J = 11 and 15 Hz, CH₂-Ind), 3.17 (dd, 1H, J = 2 and 15 Hz, CH₂-Ind), 3.09 [m, 2H, ε-H (Lys)], 3.30 (m, 1H, 5-H), 3.44 (m, 1H, 3-H), 3.47 (m, 2H, 6-H), 3.58 (d, 1H, J = 18 Hz, 4-CH₂), 3.70 (d, 1H, J = 18 Hz, 4-CH2), 4.02 (m, 1H, 5-CH), 4.64 [m, 2H, 1-CH₂ (Bn)], 5.92 [m, 1H, NHBoc (Lys)], 6.14 (d, 1H, J = 8.5 Hz, NHBoc), 7.18-7.41 (m, 7H, Ar), 7.48 (s, 1H, Ar), 7.61 (d, 1H, J = 7.5 Hz, Ar), 8.11(d, 1H, J = 7.5 Hz, Ar).
¹³C NMR (100 MHz, (CD₃)₂CO) δ (ppm): 23.9 [C_{γ} (Lys)], 25.3 (CH₂-Ind), 28.3 [6CH₃ (Boc and ^{t}Bu)], 28.6, 28.7 [6CH₃ (2Boc)], 30.8 [C_{δ} (Lys)], 35.0 [C_{β} (Lys)], 41.1 [C_{ε} (Lys)], 44.8 [C₆], 50.0 [1-CH₂ (Bn)], 51.9 (Cs-CH), 56.9 (4-CH₂), 60.9 (C₅), 65.6 (C3), 78.2, 78.8 [2C (2Boc)], 81.7 [C (^{t}Bu)], 84.0 [C (Boc)], 115.9 [CH (Ar)], 119.1 [C (Ar)],119.9, 123.3, 124.1, 125.0, 128.1, 128.8, 129.4 [9CH (Ar)], 131.7, 136.4, 138.7 [3C (Ar)], 150.3, 156.6 [3CO (3Boc)], 171.2 (CO₂), 171.3 (C₂);
ES-MS m/z 835.1 [M+1]⁺;
Anal. calcd. For C₄₆H₆₇N₅O₉: C, 66.24; H, 8.10; N, 8.40. Found: C, 65.98; H, 8.36; N, 8.61.

### Materials and methods

**Protective effect against virus-induced cytopathic effect:** Vero-E6 cells were seeded onto 96-well plates (2x10⁴ cells/well). Candidate compounds were diluted from stock solutions in complete media (DMEM) supplemented with 10 mM HEPES, IX non-essential amino acids (gibco), 100 U/mL penicillin-streptomycin (GIBCO) and 2% fetal bovine serum (heat-inactivated at 56 °C for 30 min)) to achieve the desired concentration. A wide range of compound concentrations from 50 to 0.75 µM were tested in an initial experiment.

SARS-CoV-2 stock strain NL/2020 (kindly provided by Dr. R. Molenkamp, Erasmus University Medical Center Rotterdam) was diluted to achieve a multiplicity of infection (MOI) of 0.001. Virus-compound mixture was used to inoculate the Vero-E6 monolayer. Cultures were maintained at 37°C in a 5% CO2 incubator for 72 h in the BSL3 facility. Cells were fixed using a 4% formaldehyde-PBS solution to finalize the experiment and inactivate the virus. Virus-induced cell death was revealed by staining with a 0.1% crystal violet solution in water-methanol for 30-60 minutes, time after which, plates were extensively washed with water and dried. Infected, as well as uninfected cells, were used as controls. Using crystal violet staining, the inventors determined the dilutions of compounds capable of protecting the cell monolayer from virus-induced cell death.

**Intracellular viral antigen accumulation:** To confirm that the monolayer protection effect displayed by the identified compounds was indeed directly related to an antiviral effect and not to an unrelated effect, Vero-E6 cell monolayers were inoculated at MOI 0.01 in the presence of non-toxic compound concentrations. Twenty-four hours later cells were fixed by 20 minutes incubation with a 4% formaldehyde-PBS solution. After extensive washes with PBS, infection efficiency was evaluated by the detection of intracellular SARS-CoV-2 N protein accumulation by immunofluorescence microscopy. Nuclei were stained using DAPI (4',6-diamidino-2-phenylindole) dye. Automated imaging was carried out in a SparkCyto (Tecan) multimode plate reader. The total intensity signal present in vehicle-treated conditions was used as reference (100%).

**Determination of cytotoxicity by MTT colorimetric assay:** To evaluate the cytotoxicity profile of the selected compounds MTT assays were performed in Vero-E6 cells. Uninfected cells were incubated with a range of compound concentrations (from 50 to 0.78 µM) for forty-eight hours, time at which the MTT substrate [3-(4,5-dimethylthiazol-2-yl)-2,5-diphenyltetrazolium bromide] was added and the formation of formazan precipitates was evaluated two hours later, using a colorimetric readout. Vehicle-treated cells were used as reference (100%).

**Intracellular viral RNA quantitation:** To confirm that the monolayer protection effect displayed by the identified compounds was indeed directly related to an antiviral effect and not to an indirect and unrelated effect, Vero-E6 cell monolayers were inoculated at MOI 0.001 in the presence of non-toxic compound concentrations. Forty-eight hours later cell lysates were prepared using Trizol reagent (Thermo Scientific) and the viral RNA content was determined by RT-qPCR using previously validated sets of primers and probes specific for the detection of the SARS-CoV-2 E gene and the cellular 18S gene, for normalization purposes. ΔCt method was used for relative quantitation of the intracellular viral RNA accumulation in compound-treated cells compared to the levels in infected cells treated with DMSO, set as 100%.

**Table 1. Evaluation of potency and the cytotoxicity profile for the compounds of examples 2 to 6.**

| **Example** | **EC₅₀ mean (µM)** | **S dev** | **EC₉₀ mean (µM)** | **S dev** | **CC₅₀ (µM)** |
|---|---|---|---|---|---|
| **2** | 15.33 | 10.50 | 46.67 | 1.53 | > 50 |
| **3** | 6.33 | 4.04 | 14.33 | 0.58 | > 50 |
| **4** | 1.60 | 1.22 | 14.67 | 4.16 | > 50 |
| **5** | 1.93 | 1.01 | 7.33 | 4.04 | > 50 |
| **6** | 3.17 | 1.26 | 9.67 | 4.04 | > 50 |

**Table 2. qPCR results for the compounds of examples 3 and 6.**

| **Example** | **% RNA relative to DMSO** |
|---|---|
| DMSO | 10² |
| **3** | 10⁻² |
| **6** | 10⁻⁴ |

The qPCR results indicate that the compounds of examples 3 and 6 are particularly effective antivirals at 15µM, reaching virtually undetectable levels as compared with the uninfected controls.

### Activity on recombinant human coronavirus 229E-GFP

Huh7-Lunet#3 cells (kindly provided by Dr. Thommas Pietschmann; Twincore-Hannover) were maintained subconfluent in complete media [(DMEM supplemented with 10 mM HEPES, IX non-essential amino acids (Gibco), 100 U/ mL penicillin-streptomycin (Gibco) and 10 % Fetal Bovine Serum (FBS; heat-inactivated at 56 °C for 30 min)].

Huh7-Lunet#3 cells were seeded onto 96-well plates (1x10⁴ cells/well). The day after, compound stock solutions (10 mM in DMSO) were diluted into complete cell culture media to achieve a final concentration of 20 µM. On the other hand, hCoV-229E-GFP virus (Human coronavirus 229E expressing Green Fluorescent Protein) stock (kindly provided by Dr. Volker Thiel; University of Bern) was diluted in complete media to achieve a final concentration of 3x10³ focus forming units (FFU)/ml. One hundred microliters (100 µl) of the virus dilution were mixed 1:1 with 100 µl of the compound dilution to achieve final compound concentrations of 10 µM and 150 infectious units (FFU) per well in a 96 well plate. One hundred µl of the mixture was applied onto the Huh7-Lunet #3 cell monolayer in biological replicates and cells were cultured for 72 hours at 33°C in a 5% CO₂ incubator. Cells were fixed in a 4% formaldehyde solution in PBS for 10 minutes at room temperature, washed twice with PBS and individual well fluorescence was measured in a SpectraMax iD3 fluorescence plate reader (Molecular Devices). Background subtraction was performed using non-infected wells and signal was normalized to the average fluorescence found in vehicle (DMSO)-treated virus-infected wells. Once infection efficiency had been determined, plates were stained with a 0.1% crystal violet solution in water-methanol for 30-60 minutes. Then, plates were extensively washed with water and dried before 1% SDS solution in water was added to solubilize crystal violet. Absorbance was measured at 570 nm and background was subtracted from blank wells. Relative well biomass was estimated by calculating the absorbance in each well relative to the average observed in infected cells treated with DMSO.

**Table 3. Reduction of hCoV-229E-GFP propagation by the compounds of examples 3 to 6.**

| **Compound** | % Reduction of hCoV-229E-GFP propagation |
|---|---|
| **3** | 94 |
| **4** | 97 |
| **5** | 94 |
| **6** | 98 |

Compound of examples **3** to **6** (10µM) reduced hCoV-229E-GFP propagation in the absence of measurable cytotoxicity, showing that it also displays antiviral activity against hCoV-229E-GFP.

### ABBREVIATIONS

The following abbreviations and symbols used within the present application are defined:
¹³C-NMR Carbon-13 nuclear magnetic resonance
¹H-NMR Proton nuclear magnetic resonance
BnBr Benzyl bromide
Boc tert-Butyloxycarbonyl
BSL3 Biosafety level 3
COMU (1-Cyano-2-ethoxy-2-oxoethylidenaminooxy)dimethylamino-morpholino-carbenium hexafluorophosphate
DAPI 4',6-diamidino-2-phenylindole
DBU 1,8-Diazabicyclo[5.4.0]undec-7-ene
DCM Dichloromethane
DIC Diisopropylcarbodiimide
DIEA or DIPEA N,N-Diisopropylethylamine
DMEM Dulbecco's modified Eagle's medium
DMF Dimethylformamide
DMSO Dimethylsulfoxide
EDC 1-Ethyl-3-(3-dimethylaminopropyl)carbodiimide
ESI Electrospray ionization
Et3N or TEA Triethyl amine
EtOAc Ethyl acetate
Fmoc Fluorenylmethyloxycarbonyl
HATU 1-[Bis(dimethylamino)methylene]-1H-1,2,3-triazolo[4,5-b]pyridinium 3-oxid hexafluorophosphate
HEPES 4-(2-Hydroxyethyl)-1-piperazineethanesulfonic acid
HOAt 1-Hydroxy-7-azabenzotriazole
HOBt N-Hydroxybenzotriazole
HPLC High performance liquid chromatography
Ind Indol
Lys Lysine
MeOH methanol
MS Mass spectroscopy
MTT 3-(4,5-Dimethylthiazol-2-yl)-2,5-diphenyltetrazolium bromide
NNM *N*-Methyl morpholine
PBS Phosphate-buffered saline
Py AOP 7-Azabenzotriazol-1-yloxy)tripyrrolidinophosphonium hexafluorophosphate
RT-qPCR Real-time polymerase chain reaction
THF Tetrahydrofuran
TLC Thin layer chromatography
TMSCN Trimethylsilyl cyanide

## Claims

1. A compound of formula (I) 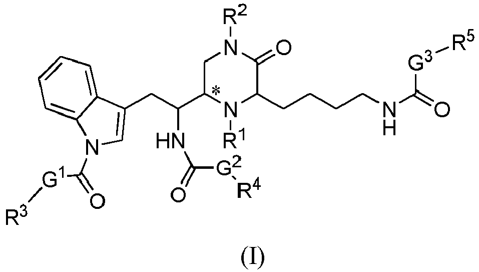 its stereoisomers and pharmaceutically acceptable salts thereof for use in the treatment and/or prevention of an infection by viruses of the family *Coronaviridae,* wherein
• R¹ is a group selected from hydrogen, benzyl and -CH₂C(O)O-R⁶,
• R² is a group selected from hydrogen and benzyl,
• R³, R⁴ and R⁵ are independently selected from the group consisting of linear or branched C₁₋₆-alkyl, 6-14 membered aryl, 6-14 membered heteroaryl comprising 1, 2 or 3 heteroatoms selected from the group consisting of N, O and S, wherein the aryl and heteroaryl groups may be substituted by 1 to 3 substituents selected from the group consisting of halogen atom, linear or branched C₁₋₃ haloalkyl, linear or branched C₁₋₃ alkyl, linear or branched C₁₋₃ alkoxy, cyano group and hydroxy group,
• G¹, G² and G³ are either absent or are independently selected from the group consisting of -O-, -O-CH₂-, wherein the oxygen atom is bound to the corresponding carbonyl group, -CH₂-, and -NH-; and
• R⁶ is selected from the group consisting of linear or branched C₁₋₆-alkyl and benzyl.

2. Compound for use according to claim 1, wherein two of the groups R³, R⁴ and R⁵ are identical.

3. Compound for use according to any one of claims 1 to 2, wherein two of the groups G¹, G² and G³ are identical.

4. Compound for use according to any one of claims 1 to 3 wherein R³-G¹-, R⁴-G²- and R⁵-G³- are independently selected from the group consisting of C₁₋₆-alkoxy and C₆₋₁₄-aryl-CH₂-O- and C₆₋₁₄-aryl-CH₂-.

5. Compound for use according to any one of claims 1 to 4 wherein R³-G¹-, R⁴-G²- and R⁵-G³- are independently selected from the group consisting *tert*-butoxy and fluorenylmethyloxy.

6. Compound for use according to any one of claims 1 to 5 wherein R² is hydrogen.

7. Compound for use according to claim 1 wherein the compound is one of:
• (5*R*,3*S*)-5-((*S*)-1-(*tert*-Butoxycarbonyl)amino-2-(1-(*tert*-butoxycarbonyl)(indol-3-yl))-ethyl)-3-(4-(*tert*-butoxycarbonyl)amino-butyl)-2-oxopiperazine
• (5*S*,3*S*)-5-((*S*)-1-(*tert*-Butoxycarbonyl)amino-2-(1-(*tert*-butoxycarbonyl)(indol-3-yl))-ethyl)-3-(4-(*tert*-butoxycarbonyl)amino-butyl)-2-oxopiperazine
• (5*R*,3*S*)-4-Benzyl-5-((*S*)-1-(*tert*-butoxycarbonyl)amino-2-(1-(*tert-*butoxycarbonyl)(indol-3-yl))-ethyl)-3-(4-(*tert*-butoxycarbonyl)amino-butyl)-2-oxopiperazine
• (5*S*,3*S*)-4-Benzyl-5-((*S*)-1-(*tert*-butoxycarbonyl)amino-2-(1-(*tert-*butoxycarbonyl)(indol-3-yl))-ethyl)-3-(4-(*tert*-butoxycarbonyl)amino-butyl)-2-oxopiperazine
• (5*R*,3*S*)-5-((*S*)-1-(*tert*-Butoxycarbonyl)amino-2-(1-(*tert*-butoxycarbonyl)(indol-3-yl))-ethyl)-4(*tert*-butoxycarbonyl)methyl-3-(4-(*tert*-butoxycarbonyl)amino-butyl)-2-oxopiperazine
• (5*S*,3*S*)-5-((*S*)-1-(*tert*-Butoxycarbonyl)amino-2-(1-(*tert*-butoxycarbonyl)(indol-3-yl))-ethyl)-4(*tert*-butoxycarbonyl)methyl-3-(4-(*tert*-butoxycarbonyl)amino-butyl)-2-oxopiperazine
• (5*R*,3*S*)-1,4-Dibenzyl-5-((*S*)-1-(*tert*-butoxycarbonyl)amino-2-(1-(*tert-*butoxycarbonyl)(indol-3-yl))-ethyl)-3-(4-(*tert*-butoxycarbonyl)amino-butyl)-2-oxopiperazine
• (5*S*,3*S*)-1,4-Dibenzyl-5-((*S*)-1-(*tert*-butoxycarbonyl)amino-2-(1-(*tert-*butoxycarbonyl)(indol-3-yl))-ethyl)-3-(4-(*tert*-butoxycarbonyl)amino-butyl)-2-oxopiperazine
• (5*R*,3*S*)-1-Benzyl-5-((*S*)-1-(*tert*-butoxycarbonyl)amino-2-(1-(*tert-*butoxycarbonyl)(indol-3-yl))-ethyl)-4-(*tert*-butoxycarbonyl)methyl-3-(4-(*tert-*butoxycarbonyl)amino-butyl)-2-oxopiperazine
• (5*S*,3*S*)-1-Benzyl-5-((*S*)-1-(*tert*-butoxycarbonyl)amino-2-(1-(*tert-*butoxycarbonyl)(indol-3-yl))-ethyl)-4-(*tert*-butoxycarbonyl)methyl-3-(4-(*tert-*butoxycarbonyl)amino-butyl)-2-oxopiperazine
and pharmaceutically acceptable salts thereof.

8. A compound for use according to any one of claims 1 to 7 wherein the infection by viruses is selected from infections by respiratory syndrome-related coronaviruses selected from the species *"Severe respiratory syndrome-related coronavirus"* and *"Middle East respiratory syndrome-related coronavirus".*

9. Compound for use according any one of claims 1 to 8, wherein the respiratory syndrome-related coronavirus is selected from the group consisting of SARS-CoV-2, SARS-CoV and MERS-CoV.

10. Compound for use according to any one of the preceding claims, wherein the viral infection is by SARS-CoV-2 viruses.

11. A pharmaceutical composition comprising a compound according to any one of claim 1 to 8 and at least one pharmaceutically acceptable excipient for use in the treatment and/or prevention of viral infections.
